Europäisches Patentamt

European Patent Office

Office européen des brevets

(19)

(11) Numéro de publication : **0 627 401 A1**

# (12) DEMANDE DE BREVET EUROPEEN

(21) Numéro de dépôt : 94401121.2

(22) Date de dépôt : 20.05.94

(51) Int. Cl.⁵ : **C07C 53/08, C07C 51/215**

(30) Priorité : 28.05.93 FR 9306475

(43) Date de publication de la demande :
07.12.94 Bulletin 94/49

(84) Etats contractants désignés :
**BE DE FR GB IT NL**

(71) Demandeur : **RHONE-POULENC CHIMIE
25, Quai Paul Doumer
F-92408 Courbevoie Cédex (FR)**

(72) Inventeur : **Blaise, François
7, Square de la Pépinière
F-94550 Cheville Larue (FR)**
Inventeur : **Bordes, Elisabeth
26, rue Léon Bouchard
F-95470 Vemars (FR)**
Inventeur : **Gubelmann, Michel
22, rue Raynouard
F-75016 Paris (FR)**
Inventeur : **Tessier, Laurent
119, rue des Pyrénées
F-75020 Paris (FR)**

(74) Mandataire : **Seugnet, Jean Louis et al
RHONE-POULENC CHIMIE,
Direction de la Propriété Industrielle,
25, Quai Paul Doumer
F-92408 Courbevoie Cédex (FR)**

(54) **Procédé de préparation d'acide acétique par oxydation par oxydation de l'éthane.**

(57)    La présente invention a trait à un procédé de préparation d'acide acétique par oxydation ménagée de l'éthane.
    Le procédé selon l'invention consiste à faire réagir l'éthane avec une source d'oxygène ; la réaction étant effectuée en présence d'un catalyseur dont la phase active est à base de vanadium (V), de titane et d'oxygène.

EP 0 627 401 A1

La présente invention a trait à un procédé de préparation d'acide acétique par oxydation ménagée de l'éthane en présence d'un catalyseur à base notamment de vanadium (V), de titane et d'oxygène.

L'obtention d'acide acétique par oxydation ménagée de l'éthane, en présence d'un catalyseur, est un procédé en plein développement car il présente l'avantage d'utiliser une matière première dont le coût est intéressant.

Parmi les procédés connus de ce type, on peut citer ceux mettant en jeu, outre l'éthane et l'oxygène, des catalyseurs complexes à base d'au moins un mélange d'oxydes de molybdène, de vanadium, de niobium et d'antimoine. Cependant, la sélectivité en acide acétique par mise en oeuvre de tels catalyseurs, est de l'ordre de 15 à 25 % seulement, alors que les conditions de réactions sont relativement dures. En effet, il est nécessaire d'effectuer l'oxydation dans des conditions combinant une température élevée, c'est-à-dire de l'ordre de 400°C, et une pression de 20 bar.

Un autre procédé consiste à faire réagir l'éthane et l'oxygène en présence d'un catalyseur à base au moins d'oxydes de vanadium, de tungstène et de rhénium. Les sélectivités en acide acétique sont améliorées par rapport à celles des catalyseurs précédemment décrits (de l'ordre de 30 %) mais restent cependant inférieures à celles de l'éthylène coproduit. En outre, ce procédé nécessite d'introduire dans le réacteur de fortes quantités d'eau et d'un diluant comme l'hélium ou l'azote.

Il est par ailleurs connu d'utiliser des catalyseurs dont la phase active comprend des oxydes de vanadium, ce dernier étant au degré d'oxydation (IV), de titane et/ou de phosphore. Dans le cas de ces catalyseurs, les performances sont intéressantes en termes de sélectivité en acide acétique. En revanche, les conditions de réactions rendent impossible l'exploitation de ce procédé à l'échelle industrielle. En effet, la conversion de l'éthane n'est pas élevée et le flux d'alimentation comprend plus de 90 % de gaz diluant, ce qui a pour résultat une productivité très faible.

Ainsi que l'on peut le constater, on ne dispose pas, à l'heure actuelle, de procédés de préparation d'acide acétique par oxydation de l'éthane pouvant être développés à l'échelle industrielle et mettant en jeu des catalyseurs de composition simple.

Or il a été trouvé de façon totalement inattendue que l'utilisation de catalyseurs à base de vanadium (V), de titane et d'oxygène, dans la réaction précitée, permettait de pallier les inconvénients mentionnés ci-dessus.

Ainsi, le procédé selon l'invention combine une sélectivité intéressante en acide acétique formé avec une productivité permettant d'envisager une exploitation de la réaction à l'échelle industrielle.

Un avantage supplémentaire du procédé selon l'invention réside dans la température très faible d'activation de l'éthane. En effet, les températures classiques nécessaires pour l'activation d'hydrocarbures dans des réactions d'oxydation, telles que l'oxydation du propylène, de l'orthoxylène, du butane sont voisines de 400°C. Sachant que les oléfines sont plus réactives que les alkylaromatiques, eux-mêmes étant beaucoup plus réactifs que les alcanes et qu'en outre, la réactivité des hydrocarbures saturés augmente avec le nombre d'atomes de carbone présents dans la molécule, il est tout à fait surprenant de pouvoir activer l'éthane à des températures pouvant être aussi faibles que 150°C-250°C.

Par ailleurs, et ceci représente un autre avantage, la période d'activation du catalyseur mis en oeuvre selon l'invention est beaucoup plus faible que pour les catalyseurs mis en oeuvre dans les procédés classiques, ceci étant dû notamment à une température d'utilisation plus faible.

Par ailleurs, le catalyseur mis en oeuvre dans le procédé selon l'invention présente l'avantage d'être une composition catalytique de formulation simple, et bien connue de l'homme de l'art, bien qu'exploitée dans des applications différentes.

Ces buts et d'autres sont atteints par la présente invention qui concerne donc un procédé de préparation d'acide acétique par réaction en phase gazeuse d'éthane et avec une source d'oxygène, en présence d'un catalyseur dont la phase active comprend du vanadium (V), du titane et de l'oxygène.

Pour plus de commodité, le catalyseur mis en oeuvre dans l'invention va tout d'abord être décrit.

Ainsi que cela a été indiqué auparavant, il a été trouvé d'une façon totalement inattendue que la mise en oeuvre de catalyseurs à base de vanadium (V) et de titane, en tant qu'éléments constitutifs de la phase active, était efficace pour l'obtention d'acide acétique à partir d'éthane.

Ainsi, la phase active du catalyseur utilisé comprend du vanadium (V). De préférence, le vanadium se présente sous forme de $V_2O_5$.

La quantité de vanadium, exprimée en $V_2O_5$, de la phase active du catalyseur est comprise entre 0,1 et 30 % en poids. Il est à noter que des teneurs en dehors de cette gamme ne sont pas exclues bien qu'elles n'apportent pas d'avantages supplémentaires.

Selon un mode plus particulier de réalisation de l'invention, le catalyseur mis en oeuvre dans la présente invention comprend une phase active dont la teneur en vanadium, exprimée en $V_2O_5$, est comprise entre 0,2 et 15 % en poids.

Le titane présent dans la phase active peut être indifféremment sous forme de $TiO_2$ anatase, rutile, broo-

2

kite ou encore bronze (symbolisé (B)), ou leurs mélanges.

De préférence, la forme allotropique de l'oxyde de titane est choisie parmi les formes anatase, rutile, ou leurs mélanges.

L'oxyde de titane entrant dans la composition de la phase catalytique présente en outre une surface spécifique, mesurée selon la méthode B.E.T., comprise entre 1 et 150 m²/g. Plus particulièrement la surface spécifique est comprise entre 10 et 120 m²/g.

Par ailleurs, la phase active du catalyseur utilisé dans la présente invention peut comprendre au moins un dopant.

Le dopant est choisi parmi les éléments suivants: K, Rb, Cs, Ca, Mg, Zr, Hf, Nb, Ta, Cr, Mo, W, Mn, Re, Fe, Ru, Os, Co, Rh, Ir, Ni, Pd, Cu, Ag, Zn, Cd, Tl, Si, Ge, Sn, P, As et Sb.

De préférence, le dopant est choisi dans la liste suivante : K, Cs, Zr, Nb, Ta, Cr, Mo, W, Mn, Re, Fe, Ru, Co, Pd, Ag, Zn, Tl, P et Sb.

Habituellement, la quantité de dopant dans la phase active est inférieure ou égale à 1,5 % atomique par rapport au vanadium.

La phase active du catalyseur mis en oeuvre dans la présente invention peut être obtenue par toute méthode connue de l'homme du métier.

On peut envisager par exemple de fabriquer ladite phase active par mélange des oxydes des éléments constitutifs de la phase active, suivi d'une étape de calcination, éventuellement suivie et/ou précédée par un broyage dudit mélange (technique de chamottage).

Une autre méthode convenable pour la préparation de la phase active consiste à effectuer les étapes suivantes :

- on prépare un mélange à base d'au moins l'un des éléments constitutifs de la phase active ;
- on précipite éventuellement lesdits éléments ;
- on sèche le mélange éventuellement traité ;
- on calcine le produit séché.

Par éléments constitutifs, on désigne non seulement le vanadium et le titane mais aussi le ou les dopants ajoutés à la composition de la phase active.

Habituellement, les éléments constitutifs sont utilisés sous la forme d'une solution ou d'une suspension.

D'une façon avantageuse, le milieu dispersant (ou solubilisant) est l'eau, bien que tout autre type de dispersant (ou solvant) soit envisageable. A ce titre, on peut citer notamment les alcools, comme le méthanol, l'éthanol, l'isopropanol, le tertiobutanol.

Les éléments constitutifs de la phase active entrant dans la composition du mélange précité, sont utilisés généralement sous forme de sels d'acides inorganiques ou organiques, ou encore sous forme de composés comme les oxydes ou leurs dérivés.

Tous les acides ou dérivés d'oxydes indiqués conviennent à la préparation du mélange dans la mesure où ceux-ci peuvent se décomposer en oxyde du ou des éléments correspondants.

A titre d'exemples de sels d'acides inorganiques convenant à la préparation du mélange précité, on peut mentionner entre autres les nitrates, les sulfates, les halogénures comprenant un ou plusieurs halogènes, les sels d'ammonium.

A titre d'exemples de sels d'acides ou d'esters organiques, on peut mentionner le formiate, l'oxalate, le tartrate, l'acétate, l'acétylacétonate, l'éthylhexanoate.

Ainsi qu'il a été indiqué auparavant, il est aussi envisageable d'utiliser des oxydes ou leurs dérivés ; ces composés pouvant être utilisés sous forme de particules, ou solubilisés, notamment par ajout d'un acide ou d'une base au dit mélange.

Par dérivés d'oxydes, on entend les composés du type des oxyhalogénures, des alkoxydes, les aryloxydes, les glycoxydes notamment.

Il est à noter que tous ces composés peuvent être utilisés seuls ou en mélange.

A titre d'exemples de composés comprenant du vanadium, convenant à la mise en oeuvre de ce mode de préparation, on peut citer, sans intention de se limiter, le sulfate de vanadyle, le métavanadate d'ammonium, les oxyhalogénures de vanadium tels que, notamment, $VOCl_3$, $(VO_2)Cl$, $VOCl$, $VOBr$, $VOBr_2$, $VOF_3$, $VOF_2$, $VF_4$, $VBr_2$, $VI_2$, l'acétylacétonate de vanadyle, l'oxalate de vanadyle, l'acide métavanadique, l'hexacarbonyle de vanadium, le tri-isopropoxyde d'oxyde de vanadium, les oxydes de vanadium comme par exemple, $V_2O_5$, $V_7O_{13}$, $VO$, $VO_2$, $V_2O_3$, $V_3O_7$.

En tant que composé comprenant du titane, on peut citer les composés du type $TiX_4$ avec X représentant un halogène et plus particulièrement le chlore, les composés du type $Ti(OR)_4$ avec R représentant un groupement alkyle et plus particulièrement les radicaux éthyle, isopropyle ou encore sec-butyle.

Convient aussi à la mise en oeuvre de l'invention l'oxyde de titane sous ses diverses formes allotropiques, c'est-à-dire sous forme anatase, rutile, brookite ou (B).

En ce qui concerne les composés à base d'un dopant, ceux-ci sont choisis parmi les chlorures de potassium, l'acétate de potassium, les chlorures de rubidium, le chlorure ou l'oxychlorure de niobium, l'oxalate de niobium, le sulfate de césium, l'acétate de césium, le sulfate de fer, l'acétate de fer, les chlorures ou chlorates de chrome, les nitrates de chrome, l'acétate de chrome, l'acétylacétonate de chrome, le dimolybdate d'ammonium, le métatungstate et le paratungstate d'ammonium, les oxydes et alkyloxydes, comme l'éthoxyde, de zirconium, l'oxyde d'argent. Bien entendu, cette liste ne peut être considérée comme limitative.

La température à laquelle le mélange des éléments constitutifs est effectué est habituellement comprise entre 20 °C et la température d'ébullition du dispersant ou solvant choisi.

De façon générale, la concentration dudit mélange varie entre 0,1 et 5 M.

Selon un premier mode de réalisation particulier, on effectue ensuite une étape de précipitation d'au moins l'un des éléments constitutifs présent sous forme solubilisée.

Lesdits éléments peuvent être précipités, soit simultanément, soit successivement. Par ailleurs, tout ou partie de ces derniers peut être précipité lors de cette opération.

Dans ce dernier cas, on effectuera les première et deuxième étapes autant de fois que nécessaire pour que le produit à sécher contienne tous les éléments constitutifs de la phase active.

Tout type d'agent précipitant convient à la mise en oeuvre de cette étape dans la mesure où il se combine à un ou plusieurs éléments constitutifs, sous la forme d'un composé insoluble dans le milieu choisi et dans la mesure où ledit composé insoluble permet d'accéder à l'oxyde du ou des éléments combinés.

Il est à noter que la précipitation peut être réalisée avec un ou plusieurs agents précipitants.

Classiquement, ledit agent est choisi, entre autres parmi les bases du type de l'ammoniaque notamment, ou parmi les acides inorganiques ou organiques, comme l'acide chlorhydrique ou encore l'acide citrique. Il est à noter que l'eau peut constituer un agent précipitant convenable à la mise en oeuvre de l'invention. Bien évidemment le choix des agents précipitants n'est pas limité à cette seule liste, donnée à titre indicatif.

Habituellement, la précipitation est mise en oeuvre sous agitation.

La température à laquelle elle est réalisée, varie généralement entre 20°C et la température d'ébullition du solvant choisi.

Enfin, la ou les étapes de précipitation peuvent si nécessaire être suivies d'étapes de mûrissement. Celles-ci consistent à laisser le précipité en suspension, sous agitation, pendant une durée variant de 30 minutes à 4 heures environ.

La température de mûrissement est comprise dans la gamme de température indiquée précédemment.

Le mélange comprenant les éléments constitutifs de la phase active, ayant subi une ou plusieurs étapes de précipitation, est ensuite séché.

Un second mode de réalisation du procédé consiste à sécher le mélange sans le soumettre préalablement à l'étape de précipitation décrite ci-dessus.

Selon une première variante, le séchage est effectué en deux temps : le premier consistant à évaporer le solvant ou dispersant du mélange, jusqu'à siccité, le second à sécher la pâte ainsi obtenue.

Généralement, la première étape est réalisée à une température variant de 20 à 100°C pendant la durée nécessaire pour obtenir une pâte non coulante.

L'évaporation est habituellement effectuée sous agitation.

La pâte résultante est ensuite séchée, dans un second temps, sous une atmosphère de préférence non réductrice, comme l'oxygène ou l'air par exemple, pendant une durée moyenne de 15 heures.

La température de séchage est habituellement d'environ 120°C.

Selon une seconde variante, le séchage est effectué en atomisant la solution ou suspension, par tout moyen connu de l'homme du métier. Ainsi, sans intention de se limiter, les atomiseurs de type BUCHI ou encore les atomiseurs de type "flash" comme revendiqués dans les demandes de brevets français publiées sous les numéros suivants : 2 257 326, 2 419 754, 2 431 321, conviennent à ce mode de réalisation.

La température d'atomisation est généralement de l'ordre de 150 à 300°C.

L'atmosphère sous laquelle est effectuée l'atomisation, là encore, est de préférence non réductrice. De façon avantageuse, on effectue l'atomisation sous air, bien que l'oxygène soit envisageable pour une telle étape.

Le produit séché obtenu selon chacune des variantes précitées, est ensuite soumis à une étape de calcination.

Celle-ci est réalisée, de façon classique, sous une atmosphère non réductrice. Avantageusement on utilise l'air, mais l'oxygène pourrait tout aussi bien être employé.

La température de calcination est habituellement comprise entre 200 et 1200°C.

Généralement, la durée de l'opération varie entre 1 et 24 heures.

Préalablement à l'étape de calcination, le produit séché peut subir une étape de broyage.

Il est à noter que le produit calciné peut éventuellement subir aussi un tel traitement.

Le catalyseur mis en oeuvre dans le procédé selon la présente invention peut comprendre essentiellement la phase active décrite ci-dessus (forme massique) ou comprendre en outre un diluant (forme diluée).

Dans le cas particulier où le catalyseur comprend un diluant (ou support), la phase active peut être soit déposée sur celui-ci, soit l'enrober ou encore y être mélangée.

La nature du diluant n'est pas critique, si ce n'est qu'il doit être inerte vis-à-vis des réactifs dans les conditions réactionnelles choisies.

A titre de matériaux susceptibles d'être utilisés comme support de catalyseur, on peut citer : la silice, l'alumine, la silice-alumine, l'argile frittée, la magnésie, le silicate de magnésium, la terre de diatomée. Ces types de support peuvent être utilisés sous forme poreuse ou non. De préférence, le support utilisé est sous forme non poreuse. Si nécessaire, on peut effectuer un émaillage de ceux-ci afin de le rendre tel.

Les matériaux céramiques, du type de la cordiérite, l'alumine, la mullite, la porcelaine, le nitrure de silicium, les carbures de bore et de silicium, peuvent aussi être utilisés comme diluant.

Le catalyseur mis en oeuvre dans le procédé selon l'invention, dilué ou non, se présente sous forme de particules ou de monolithe.

Dans le cas où le catalyseur est constitué de particules, la granulométrie de celles-ci dépend du mode d'utilisation du catalyseur. Elle peut donc varier dans de larges limites, comme notamment être comprise entre quelques micromètres et une dizaine de millimètres. Plus particulièrement, et à titre indicatif, un catalyseur utilisé en lit fixe, présente une répartition granulométrique généralement comprise entre 0,5 et 6 mm. La granulométrie des particules d'un catalyseur utilisé en lit fluidisé ou mobile, est habituellement comprise entre 5 et 700 microns, et de préférence comprise entre 5 et 200 microns pour 80 % des particules.

D'une façon classique, la quantité de diluant entrant dans la composition du catalyseur varie dans de larges limites, dépendant, la plupart du temps, du mode de mise en forme du catalyseur.
Ainsi, les catalyseurs obtenus par enrobage ou dépôt de la phase active sur le support présentent une quantité de phase active variant habituellement entre 0,1 et 30 % et de préférence entre 2 et 20 % en poids total de catalyseur fini (phase active et support). Dans les cas où le catalyseur comprend d'un support dispersé dans la phase active, la quantité de phase active est comprise entre 1 et 90 % en poids total de catalyseur fini.

Selon un mode de réalisation particulier de l'invention, la réaction est réalisée en présence d'un catalyseur de type enrobé.

Le catalyseur selon l'invention peut être obtenu selon toute méthode classique connue de l'homme du métier.

Ainsi, les catalyseurs massiques, comprenant essentiellement la phase active telle que définie plus haut, peuvent être mis en forme par extrusion, par moulage, par broyage, concassage ou tout autre moyen, de la phase active ou de son précurseur, de façon à donner un monolithe ou encore des particules de granulométrie convenable.

Ici et pour tout le reste de la description, on entend par précurseur de la phase active, le mélange des éléments constitutifs de cette phase dans tous les états antérieurs à l'étape de calcination décrite auparavant.

Dans le cas de catalyseurs comprenant un diluant, les moyens décrits précédemment sont utilisables. Ainsi, on peut mélanger la phase active avec la proportion requise de diluant, et par exemple, extruder ou mouler le mélange résultant.

Cependant d'autres méthodes sont envisageables.

Ainsi, selon un premier mode de réalisation, on met en contact le diluant, de préférence sous forme de particules rugueuses, et la phase active ou son précurseur, dans un mélangeur à fort cisaillement (appareils type LODIGE) ou dans un appareil de granulation (drageoirs, sous forme de tambour ou assiette).

L'opération est effectuée en général à une température variant entre 20 et 150°C pendant la durée nécessaire à l'enrobage du support par la quantité désirée de phase active, plus particulièrement sous air, pendant au moins 30 minutes.

Les particules ainsi obtenues sont habituellement calcinées à une température comprise entre 300 et 600°C, de préférence entre 450 et 500°C.

La durée de la calcination est généralement d'au moins 3 heures.

Un second mode possible de fabrication du catalyseur consiste à mettre en application la technique d'imprégnation.

Selon cette technique, on imprègne le support avec une suspension de la phase active ou avec une suspension ou solution de son précurseur.

L'étape d'imprégnation est suivie d'une étape de séchage, habituellement effectuée à une température comprise entre 100 et 200°C, sous air, pendant au moins 30 minutes.

On peut ensuite renouveler le cycle imprégnation - séchage et terminer celui-ci par une calcination sous air.

La température de calcination est comprise entre 300 et 600°C pendant une dizaine d'heures.

Une variante possible consiste à effectuer une calcination entre le ou les cycles imprégnation-séchage.

Selon un troisième mode de préparation du catalyseur, on ajoute au mélange d'au moins l'un des éléments constitutifs de la phase active, le support, préférentiellement sous forme de particules. Le mélange ainsi obtenu est ensuite traité conformément aux divers modes de réalisation du procédé de préparation de la phase catalytique, tels que décrits auparavant.

Bien entendu, tous ces modes de préparation ne sont donnés qu'à titre indicatif et ne peuvent en aucun cas constituer une liste exhaustive.

La présente invention concerne par ailleurs un procédé de préparation d'acide acétique par réaction en phase gazeuse, d'éthane avec une source d'oxygène, en présence d'un catalyseur tel que défini précédemment.

Il n'y a pas de conditions particulières concernant la qualité de l'éthane mis en oeuvre. Cependant, pour des questions évidentes de séparation de l'acide formé, on préfère utiliser de l'éthane présentant une pureté d'au moins 90 %.

Ce dernier peut être utilisé indifféremment seul ou dilué dans un gaz diluant, inerte dans les conditions de la réaction. Les gaz rares, comme notamment l'hélium ou l'argon, ou encore l'azote sont des gaz diluants convenant à la mise en oeuvre du procédé selon l'invention.

La réaction d'oxydation ménagée de l'éthane est mise en oeuvre en présence d'une source d'oxygène. Celle-ci peut être à base d'oxygène pur ou dilué dans un gaz inerte. Ainsi on peut effectuer la réaction d'oxydation en utilisant de l'air comme source d'oxygène.

Selon un mode de réalisation particulier de la présente invention, le rapport molaire entre l'éthane et l'oxygène est inférieur à 20. Plus particulièrement, ce rapport est compris entre 0,01 et 0,2 ou entre 0,6 et 15.

Selon un mode préféré de l'invention, ledit rapport est compris entre 0,6 et 15.

Une variante du procédé consiste à utiliser un mélange gazeux comprenant, outre les autres constituants, de l'eau.

La composition du mélange gazeux, soit l'éthane, la source d'oxygène, le cas échéant le gaz diluant et l'eau, peut varier dans de larges limites.

Sauf mention contraire, tous les pourcentages indiqués par la suite, sont exprimés en nombre de moles total du mélange gazeux.

D'une façon générale, la teneur en éthane dans le mélange gazeux, est comprise entre 0,1 et 99,9%.

Selon un mode particulier de réalisation de l'invention, la composition du mélange gazeux est telle qu'elle se trouve en dehors de la zone d'explosivité dudit mélange.

Plus particulièrement, et afin d'avoir un mélange gazeux dont la composition se trouve commodément en dehors du domaine d'explosivité, ladite teneur en éthane est comprise entre 0,1 et 3 % ou entre 10 et 99 %.

Préférentiellement, la teneur en éthane dans le mélange gazeux précité est comprise entre 10 et 99 %.

La teneur en oxygène dans le mélange gazeux mis en oeuvre varie de même dans une large gamme de concentration. Elle est en effet comprise entre 0,1 et 99,9 %.

Selon un mode de réalisation plus particulier, la teneur en oxygène dans le mélange gazeux varie entre 1 et 90 % ou entre 97 et 99,9%.

De préférence, l'oxygène contenu dans ledit mélange est comprise entre 1 et 90%.

La teneur en eau dans le mélange gazeux mis en oeuvre est comprise entre 0 et 70%.

Selon un mode de réalisation particulier, la teneur en eau dans le mélange précité est de 0 à 20%.

La teneur en gaz diluant dans le mélange, varie habituellement entre 0 et 70%.

Plus particulièrement le mélange comprend entre 0 et 20 % de gaz diluant.

Le mélange gazeux est donc mis au contact du catalyseur selon l'invention.

Le dispositif dans lequel le procédé selon l'invention est mis en oeuvre fait partie des dispositifs classiques pour les réactions catalytiques en phase gaz ; ces derniers pouvant être utilisés en continu ou en discontinu.

Ainsi, on peut effectuer la réaction en présence d'un catalyseur en lit fixe, fluidisé ou encore transporté.

La température de réaction est en général située entre 100 et 400°C, de préférence entre 150 et 350°C.

La pression totale du mélange gazeux réactionnel varie en général entre 0,1 et 20 bar absolus.

Le débit gazeux est fixé de telle sorte que le temps de contact, calculé dans les conditions normales de température et pression, soit compris entre 0,1 et 30 secondes. De préférence, le temps de contact est compris entre 0,5 et 20 secondes. Il est rappelé que le temps de contact correspond au rapport entre le volume du réacteur et le débit total des gaz.

L'acide acétique formé est séparé des sous produits ou des réactifs, classiquement par refroidissement puis condensation d'un mélange acide/eau. Les composés restés sous forme gazeuse, plus particulièrement l'éthane, peuvent être recyclés au réacteur, après avoir subi éventuellement une étape de séparation.

Des exemples concrets mais non limitatifs de l'invention vont maintenant être présentés.

**EXEMPLE 1**

Préparation du catalyseur $V_2O_5$. $TiO_2$/argile selon l'invention (catalyseur. A)

90 g de $TiO_2$ (soit 1,125 mol), présentant une surface BET égale à 86 m²/g (commercialisé par Rhône-Poulenc) sont introduits dans un drageoir et imprégnés à sec par 100 cm3 d'une solution d'oxalate de vanadyle.

La solution utilisée comprend 15,23 g d'acide oxalique dihydrate (soit 120,8 mmol) et 6,77 g de $V_2O_5$ commercial de JANSSEN (soit 37,2 mmol) dans l'eau.

Le produit ainsi obtenu est calciné à l'air à 500°C, pendant 3 heures.

La composition du produit calciné présente 7% en poids de $V_2O_5$ et un rapport V / Ti de 0,066.

25 g de la phase active ainsi préparée sont déposés sur 100 g de billes d'argile d'un diamètre moyen de 5 mm (commercialisé par Rhône-Poulenc) par enrobage au drageoir, à l'aide d'une solution aqueuse de glucose.

Le produit est ensuite calciné sous air à 500°C, pendant 3 heures.

La teneur finale en phase active du catalyseur est de 17,4 % en poids.

**EXEMPLE 2 COMPARATIF**

Préparation du catalyseur $V_2O_5$/argile (catalyseur. B)

1,5 g de $V_2O_5$ commercial de JANSSEN sont déposés sur 100 g de billes d'argile d'un diamètre moyen de 5 mm (commercialisé par Rhône-Poulenc) par enrobage au drageoir, à l'aide d'une solution aqueuse de glucose.

Le produit est ensuite calciné sous air à 500°C, pendant 3 heures.

La teneur finale en phase active du catalyseur est de 1,41 % en poids.

**EXEMPLE 3 COMPARATIF**

Préparation du catalyseur $TiO_2$/argile (catalyseur. C)

25 g de $TiO_2$, présentant une surface BET égale à 86 m²/g (commercialisé par Rhône-Poulenc) sont enrobés au drageoir sur 100 g de billes d'argile d'un diamètre moyen de 5 mm (commercialisé par Rhône-Poulenc) à l'aide d'une solution aqueuse de glucose.

Le produit est ensuite calciné sous air à 500°C, pendant 3 heures.

La teneur finale en phase active du catalyseur est de 16,5 % en poids.

**EXEMPLE 4 COMPARATIF**

Préparation du catalyseur $(VO)_2P_2O_7$/argile (catalyseur D)

100 g de $V_2O_5$ (soit 550 mmol) sont dissous dans un mélange d'alcools isobutylique (600 cm³) et benzylique (400 cm³). L'opération est effectuée au reflux (118°C) sous agitation pendant 2 à 3 heures.

A la dissolution complète du $V_2O_5$, on augmente la vitesse d'agitation et l'on introduit 145,8 g d'acide phosphorique à 85 % en poids (soit 1,27 mol) goutte-à-goutte, et l'on poursuit le reflux pendant 2 heures.

On récupère, après refroidissement du mélange ainsi obtenu, un précipité bleu. Ce dernier est filtré sur verre fritté puis séché à l'étuve à 110 °C pendant 3 heures.

Le produit résultant consiste en $(VO)HPO_4.0,5H_2O$.

On effectue ensuite une calcination sous azote à 400°C pendant 16 heures.

La détermination aux rayons X de la composition obtenue montre qu'elle correspond à la phase $(VO)_2P_2O_7$.

3 g de la phase ainsi préparée sont enrobés au drageoir selon la procédure indiquée dans les exemples précédents.

Le solide est ensuite séché sous air à 110°C; pendant 6 heures.

La teneur finale du catalyseur en phase active est de 3,01 % en poids.

Avant engagement dans la réaction d'oxydation de l'éthane, le catalyseur est calciné dans le réacteur, sous air, pendant 3 heures à 500°C.

**EXEMPLE 5**

Cet exemple illustre l'application des catalyseurs précédemment préparés, dans un procédé de préparation d'acide acétique à partir d'éthane.

Le catalyseur (20 cm³) est introduit dans un réacteur continu en INOX et à lit fixe, équipé d'un chauffage par bain de sable fluidisé et de deux chromatographes en ligne, l'un fonctionnant avec un détecteur à ionisation de flamme et l'autre d'un détecteur thermoconductimétrique.

Les gaz d'alimentation sont contrôlés par des débitmètres massiques.

L'eau est introduite sous forme liquide (pompe doseuse GILSON) dans un vaporiseur intégré au circuit gazeux.

Le flux d'alimentation est constitué de :

$C_2H_6/O_2/N_2/H_2O$ = 79,5/5,2/6,1/9,2 % mol.

Le débit total est de 69,17 l/h dans les conditions normales de température et de pression.

La vitesse volumique horaire (VVH) est de 3 500 h⁻¹, ce qui correspond à un temps de contact d'environ 1 seconde.

La pression du réacteur est maintenue constante à 11 bar (pression absolue).

On fait varier la température entre 250 et 300°C.

Le tableau suivant rassemble les résultats obtenus.

Dans celui-ci, les performances sont calculées de la manière suivante :

- <u>conversion de l'éthane (% mol)</u>

$$\text{conv. éthane} = \frac{(\text{nbre mole éthane entré} - \text{nbre mole éthane sorti})}{(\text{nbre mole éthane entré})} \times 100$$

- <u>conversion de l'oxygène (% mol)</u>

$$\text{conv. } O_2 = \frac{(\text{nbre mole } O_2 \text{ entré} - \text{nbre mole } O_2 \text{ sorti})}{(\text{nbre mole } O_2 \text{ entré})} \times 100$$

- <u>sélectivité d'un produit X (acide acétique, éthylène) (% mol)</u>

$$\text{sél. } X = \frac{\text{nbre mole éthane converti en X}}{(\text{nbre mole éthane entré} - \text{nbre mole éthane sorti})} \times 100$$

## <u>TABLEAU 1</u>

### <u>Récapitulatif des résultats d'oxydation ménagée de l'éthane.</u>

| Catalyseur | T (°C) | Conv. (%) | | Sél. (%) | |
|---|---|---|---|---|---|
| | | C₂* | O₂ | AcOH | C₂H₄ |
| A | 250 | 0,5 | 13 | 29 | 23 |
| | 260 | 0,9 | 26 | 27 | 21 |
| | 280 | 3,3 | 99 | 12 | 20 |
| B | 280 | 0 | | | |
| C | 280 | 0,1 | | 0 | 33 |
| D | 280 | 0 | | | |

\* La conversion maximale théorique de l'éthane en acide en fonction du rapport molaire initial et de la stoechiométrie est égale à 4,4 %

**Revendications**

1/ Procédé de préparation d'acide acétique par réaction en phase gazeuse d'éthane avec une source d'oxygène, caractérisé en ce que l'on effectue la réaction en présence d'un catalyseur dont la phase active est à base de vanadium (V) de titane et d'oxygène.

2/ Procédé selon la revendication précédente, caractérisé en ce que l'on utilise un catalyseur dont la phase active comprend du titane sous forme de $TiO_2$ anatase, rutile, brookite, bronze, ou leurs mélanges.

3/ Procédé selon l'une des revendications précédentes, caractérisé en ce que l'on utilise un catalyseur dont la phase active comprend du titane présentant une surface spécifique, mesurée selon la méthode B.E.T., comprise entre 1 et 150 m²/g et de préférence entre 10 et 120 m²/g.

4/ Procédé selon l'une quelconque des revendications précédentes, caractérisé en ce que l'on utilise un catalyseur dont la phase active comprend de 0,1 à 30 % en poids de vanadium, exprimée en $V_2O_5$, et de préférence comprend de 0,2 à 15 % en poids.

5/ Procédé selon l'une quelconque des revendications précédentes, caractérisé en ce que l'on utilise un catalyseur sous forme massique ou diluée.

6/ Procédé selon la revendication précédente, caractérisé en ce que l'on utilise un catalyseur enrobé comprenant 0,1 à 30 % en poids de phase active, par rapport au poids total de catalyseur fini, et de préférence, de 2 à 20 % en poids.

7/ Procédé selon la revendication 5, caractérisé en ce que l'on utilise un catalyseur comprenant un support dispersé dans la phase active, comprenant 1 à 90 % de phase active par rapport au poids total du catalyseur fini.

8/ Procédé selon l'une quelconque des revendications précédentes, caractérisé en ce que l'on utilise un catalyseur dont la phase active comprend au moins un dopant choisi parmi les éléments suivants : K, Rb, Cs, Ca, Mg, Zr, Hf, Nb, Ta, Cr, Mo, W, Mn, Re, Fe, Ru, Os, Co, Rh, Ir, Ni, Pd, Cu, Ag, Zn, Cd, Tl, Si, Ge, Sn, P, As et Sb.

9/ Procédé selon la revendication précédente, caractérisé en ce que la teneur en dopant est inférieure ou égale à 1,5 % atomique par rapport au vanadium.

10/ Procédé selon l'une quelconque des revendications précédentes, caractérisé en ce que l'on effectue la réaction avec un mélange gazeux comprenant 0,1 à 99,9 % en mole d'éthane, et plus particulièrement ledit mélange comprend entre 0,1 et 3 % ou entre 10 et 99 % en mole d'éthane.

11/ Procédé selon l'une quelconque de revendications précédentes, caractérisé en ce que l'on utilise comme source d'oxygène de l'air ou de l'oxygène.

12/ Procédé selon la revendication précédente, caractérisé en ce que l'on effectue la réaction avec un mélange gazeux comprenant 0,1 à 99,9 % en mole d'oxygène, et plus particulièrement comprenant entre 1 et 90 % ou entre 97 et 99,9 % en mole d'oxygène.

13/ Procédé selon l'une quelconque des revendications précédentes, caractérisé en ce que l'on utilise un mélange gazeux présentant un rapport molaire éthane/oxygène inférieur à 20 et plus particulièrement compris entre 0,01 et 0,2 ou entre 0,6 et 15.

14/ Procédé selon l'une quelconque des revendications précédentes, caractérisé en ce que l'on utilise un mélange gazeux comprenant de l'eau.

15/ Procédé selon la revendication précédente, caractérisé en ce que l'on utilise un mélange gazeux comprenant 0 à 70 % en mole d'eau, et de préférence comprenant 0 à 20 % en moles d'eau.

16/ Procédé selon l'une quelconque des revendications précédentes, caractérisé en ce que l'on utilise un mélange gazeux comprenant un gaz diluant choisi parmi les gaz rares ou l'azote.

17/ Procédé selon la revendication précédente, caractérisé en ce que l'on utilise un mélange gazeux comprenant 0 à 70 % en mole de gaz diluant et de préférence comprenant 0 à 20 % en moles dudit gaz.

18/ Procédé selon l'une quelconque des revendications précédentes, caractérisé en ce que l'on effectue la réaction à une température comprise entre 100 et 400°C, de préférence entre 150 et 350°C.

19/ Procédé selon l'une quelconque des revendications précédentes, caractérisé en ce que l'on effectue la réaction à une pression comprise entre 0,1 et 20 bar absolus.

20/ Procédé selon l'une quelconque des revendications précédentes, caractérisé en ce que l'on effectue la réaction dans des conditions telles que le temps de contact, calculé dans les conditions normales de température et pression, soit compris entre 0,1 et 30 secondes et de préférence entre 0,5 et 20 secondes.

**Office européen des brevets**

# RAPPORT DE RECHERCHE EUROPEENNE

Numero de la demande

EP 94 40 1121

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| Y | PATENT ABSTRACTS OF JAPAN vol. 7, no. 56 (C-155) (1201) 8 Mars 1983 & JP-A-57 207 545 (MITSUBISHI KASEI KOGYO K.K.) 20 Décembre 1982 * abrégé * | 1,8 | C07C53/08 C07C51/215 |
| Y | FR-A-1 585 741 (B.A.S.F.) * revendications 1-6 * | 1,8 | |
| Y | FR-A-1 480 078 (B.A.S.F.) * revendications 1-5 * | 1,8 | |

DOMAINES TECHNIQUES RECHERCHES (Int.Cl.5)

C07C
C07B

Le présent rapport a été établi pour toutes les revendications

| Lieu de la recherche | Date d'achèvement de la recherche | Examinateur |
|---|---|---|
| LA HAYE | 5 Septembre 1994 | Klag, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)